Europäisches Patentamt

European Patent Office

Office européen des brevets

(19)

(11) Veröffentlichungsnummer: **0 111 884**
**B1**

(12) **EUROPÄISCHE PATENTSCHRIFT**

(45) Veröffentlichungstag der Patentschrift:
15.10.86

(21) Anmeldenummer: 83112557.0

(22) Anmeldetag: 14.12.83

(51) Int. Cl.⁴: **C 07 D 295/02**

(54) Verfahren zur Herstellung von Hexamethylenimin.

(30) Priorität: 23.12.82 DE 3247751

(43) Veröffentlichungstag der Anmeldung:
27.06.84 Patentblatt 84/26

(45) Bekanntmachung des Hinweises auf die Patenterteilung:
15.10.86 Patentblatt 86/42

(84) Benannte Vertragsstaaten:
DE FR GB IT

(56) Entgegenhaltungen:
DE-A-2 752 307
DE-A-2 837 290
DE-B-1 117 583
FR-A-860 398

(73) Patentinhaber: Ruhrchemie Aktiengesellschaft,
Bruchstrasse 219, D-4200 Oberhausen 13 (DE)

(72) Erfinder: Horn, Gerhardt, Dr. Dipl.- Chem.,
Bunsenstrasse 19, D-4200 Oberhausen 11 (DE)
Erfinder: Frohning, Carl Dieter, Dr. Dipl.- Chem.,
Elsenbruch 1, D-4200 Oberhausen 11 (DE)
Erfinder: Liebern, Hans, Auf dem Dudel 15, D-4330
Mülheim/Ruhr (DE)

(74) Vertreter: Reichelt, Karl- Heinz, Dr., m. Br.
Ruhrchemie Aktiengesellschaft Abt. PLD
Postfach 13 01 60, D-4200 Oberhausen 13 (DE)

EP 0 111 884 B1

## Beschreibung

Die vorliegende Erfindung betrifft ein Verfahren zur Herstellung von Hexamethylenimin durch katalytische Hydrierung von ε-Caprolactam in der Gasphase.

Hexamethylenimin ist ein wertvolles Zwischenprodukt, das in der chemischen Technik vielfältige Anwendung findet, z.B. zur Herstellung von Pestiziden, Repellents, Pharmazeutika, Korrosinsinhibitoren und Stabilisatoren für Polymere.

Zur Herstellung von Hexamethylenimin im industriellen Maßstab geht man von ε-Caprolactam aus, das entweder in flüssiger oder in dampfförmiger Phase hydriert wird. Nach einem in der DE—A—24 59 547 beschriebenen Verfahren wird die Hydrierung von ε-Caprolactam in flüssiger Phase in Gegenwart von Nickel- oder Kobaltkatalysatoren und von Lösungsmitteln durchgeführt. Als Lösungsmittel werden Diethylenglykolether, Dioxan und Dodekan genannt. Wegen der relativ geringen Reaktionsgeschwindigkeit und der geringen ε-Caprolactamkonzentration in den verwendeten Lösungen — nach den Beispielen der DE—A—24 59 547 enthalten sie maximal 20 Gew.% ε-Caprolactam erfordert das beschriebene Verfahren ein großes Reaktorvolumen, das seiner wirtschaftlichen Durchführung entgegensteht.

Entsprechend den Angaben der US—A—40 14 867 können auch linear oder cyclinsche Äther als Lösungsmittel und Kupferchromit als Katalysator für die Hydrierung des ε-Caprolactams in flüssiger Phase eingesetzt werden. Als nachteilig bei diesem Verfahren erweisen sich der niedrige Umsatz von maximal 53% und der hohe spezifische Katalysatoreinsatz, der 5 bis 20 Gew.%, bezogen auf die Einsatzlösung, beträgt.

Nach einem in der DE—A—27 52 307 beschriebenen Verfahren leitet man dampfförmiges ε-Caprolactam und Wasserstoff über Katalysatoren, die Metalle der I. und II. Gruppe des Periodensystems enthalten. Vor der Hydrierung werden die Katalysatoren mit aliphatischen oder cycloaliphatischen Kohlenwasserstoffen mit 5 bis 8 Kohlenstoffatomen behandelt. Zur Lebensdauer der Katalysatoren finden sich in dieser Veröffentlichung keine Angaben.

Ebenfalls in Gegenwart von Wasserstoff in der Gasphase und an kupfer- oder kobalthaltigen Katalysatoren erfolgt die ε-Caprolactam-Hydrierung nach der Arbeitsweise der DE—A—28 37 290. Eine befriedigende Selektivitä wird hier nur dann erreicht, wenn nicht mehr als etwa 2/3 des eingesetzten ε-Caprolactams umgesetzt werden. Die Lebensdauer der Katalysatoren ist gering. Darüber hinaus ist die Gewinnung des nicht umgesetzten ε-Caprolactams aufwendig und damit ebenfalls nachteilig.

Außerdem ist in der FR—A—860 398 ein Verfahren zur Herstellung von Hexamethylenimin beschrieben, welches sich auf dem Einsatz von Caprolactam bzw. Ammoniak stützt, jedoch mit Drücken von 280 Kg/cm² in der Gasphase und 10 Äquivalenten Wasserstoff pro 8 Äquivalenten Ammoniak arbeitet.

Aufgrund der den bekannten Verfahren anhaftenden Nachteile bestand dahher die Aufgabe, ein Verfahren zur Herstellung von Hexamethylenimin aus ε-Caprolactam zu entwickeln, das auch bei hohem ε-Caprolactam-Umsatz mit hoher Selektivität zu Hexamethylenimin führt und eine hohe Lebensdauer der Katalysatoren sicherstellt.

Erfindungsgemäß wird diese Aufgabe durch ein Verfahren zur Herstellung von Hexamethylenimin gelöst, bei dem ε-Caprolactam mit Wasserstoff in der Gasphase an einem als Festbett oder als Wirbelschicht angeordneten Katalysator umgesetzt wird. Es ist dadurch gekennzeichnet, daß die katalytische Hydrierung des ε-Caprolactams in Gegenwart eines Wasserstoff-Ammoniak-Gemisches mit 0,5 bis 20 Vol.% Ammoniak, bezogen auf das Wasserstoff-Ammoniak-Gemisch, erfolgt und zwar unter den nachstehend geschilderten Bedingungen.

Überraschenderweise hat sich herausgestellt, daß durch den Zusatz von Ammoniak nicht nur die Selektivität hinsichtlich der Bildung von Hexamethylenimin, sondern auch das Standzeitverhalten des eingesetzten Katalysators entscheidend verbessert wird. Während nach bekannten Verfahren die Leistung der Katalysatoren bereits nach etwa 10 Tagen Betriebszeit deutlich abfällt, ist nach der neuen Arbeitsweise selbst nach 3-monatiger Betriebszeit kein Nachlassen der Katalysatorleistung festzustellen.

Als weiterer Vorteil des erfindungsgemäßen Verfahrens hat sich erwiesen, daß die spezifische Katalysatorbelastung, d.h. das Volumen-Verhältnis von flüssigem ε-Caprolactam zu Katalysator je Zeiteinheit gegenüber den bekannten Verfahren bedeutend gesteigert werden kann.

In der praktischen Durchführung des neuen Prozesses werden verdampftes ε-Caprolactam, Wasserstoff und gasförmiges Ammoniak zusammen über den Katalysator geleitet. Die Konzentration des Ammoniaks im Waserstoff-Ammoniak-Gemisch beträgt 0,5 bis 20 Vol.%, vorzugsweise 1 bis 5 Vol.%.

Nach dem erfindungsgemäßen Verfahren können alle Katalysatoren eingesetzt werden, die üblicherweise für die Hydrierung von ε-Caprolactam zu Hexamethylenimin in der Gasphase verwendet werden. Hierzu gehören insbesondere Kupfer-Trägerkatalysatoren, die außerdem noch Kobalt, Eisen, Nickel, Palladium, Silber, Zinkoxid, Manganoxid, Molybdänoxid, Titanoxid oder Zirkondioxid enthalten können. Besonders geeignet sind Kupfer-Trägerkatalysatoren mit 15 bis 85 Gew.% Kupfer und gegebenenfalls mit Zusätzen von Magnesium und Chrom, deren Herstellung nach dem Verfahren der DE—C—25 38 253 erfolgte.

Je nach Aktivität der eingesetzten Katlysatoren führt man die Hydrierung bei Temperaturen zwischen 200 und 350°C, vorzugsweise zwischen 220 bis 300°C und bei Atmosphärendruck oder bei Überdruck bis 10 bar, vorzugsweise bis 5 bar, durch.

Um Kapillarkondensation des ε-Caprolactams oder der Reaktionsprodukte im Porensystem der Katalysatorkörper zu vermeiden, sind relative hohe Volumverhältnisse von Wasserstoff-Ammoniak-Gemischh zu ε-Caprolactamdampf einzuhalten, z.B. 50 bis 500 : 1. Dadurch wird vermieden, daß der Partialdruck des dampfförmigen ε-Caprolactams oder der Reaktionsprodukte den für die Kapillarkondensation kritischen Bereich erreicht.

Zur Erzielung möglichst hoher ε-Caprolactam-Umsätze sind Verweilzeiten von 2 bis 20 Sekunden einzustellen. Unter Verweilzeit wird hier die durchschnittliche Aufenthaltsdauer der dampf- bzw. gasförmigen Reaktanten unter den Reaktionsbedingungen in dem vom Katalysator eingenommenen Reaktorvolumen vestanden. Bei konstant gehaltenem ε-Caprolactam-Einsatz kann die Verweilzeit über das Volumen des zugespeistenn Wasserstoff-Ammoniak-Gemisches reguliert werden.

Das nach Abscheidung der Reaktionsprodukte verbleibende Wasserstoffe-Ammoniak-Gemisch kann nach Ergänzung von Verlusten erneut dem Reaktor zugeführt werden.

Das erfindungsgemäße Verfahren ermöglicht eine spezifische Katalysatorbelastung von mehr als 0,2 Liter flüssiges ε-Caprolactam je Liter Katalysator und Stunde. Damit ist die Katalysatorbelastung gegenüber den bekannten Verfahren merklich verbessert. Außerdem vermindert der erfindugsgemäße Zusatz von Ammoniak die Bildung von Nebenprodukten.

Das neue Verfahren wird durch die nachfolgenden Beispiele näher erläutert.

### Beispiel 1

In einem mit beheizbarem Mantel ausgerüsteten Stahlrohr von 1500 mm Länge und 20 mm Durchmesser werden 150 ml eines nach den Angaben der DE—B—25 38 253 hergestellten Kupfer-Trägerkatalysators mit einem Gehalt von 60 Gew.% Cu und 20 Gew.% SiO$_2$, bezogen auf den vorreduzierten Katalysator, in Form zylinderförmiger Tabletten von 5 mm Höhe und 6 mm Durchmesser eingesetzt. Oberhalb der Katalysatorschicht wird eine 35 cm hohe Schüttung aus Glasringen angeordnet, die als Verdampfungs- und Vorwärmzone dient. Der Katalysator wird vor Beginn der Hydrierung mit einem N$_2$/H$_2$-Gasgemisch, das 3 % H$_2$ enthält, bei 160°C innerhalb von 24 Stunden reduziert, wobei der N$_2$/H$_2$-Volumenstrom 1000 V$_{Kat}$/h beträgt. In einem beheizbaren Dosierbehälter, der sich unmittelbar vor dem Eintritt des Reaktors befindet, wird das ε-Caprolactam geschmolzen. Je Stunde werden 30 ml der Schmelze (entsprechend 30 g bei der Temperatur der Schmelze von 85°C) in das auf 245°C erhitzte Reaktorrohr dosiert. Gleichzeitig leitet man 150 l/h Waserstoff und 2 l/h NH$_3$ unter einem Druck von 3 bar durch den Reaktor. Das ε-Caprolactam wird im H$_2$/NH$_3$-Strom vor Erreichen der Katalysatorzone vollständig verdampft. Das den Reaktor verlassende gasförmige Gemisch wird in einem dem Reaktor nachgeschalteten Kühler kondensiert. Das anfallende Kondensat enthält

72 Gew.-% Hexamethylenimin
11 Gew.-% nicht umgesetztes ε-Caprolactam
4 Gew.-% Nebenprodukte
13 Gew.-% Reaktionswasser.

Auch nach 35 Tagen kontinuierlicher Versuchsdurch führung ist kein Nachlassen der Katalysatorleistung zu beobachten. ε-Caprolactam-Umsatz und Produktzusammensetzung zeigen während dieser Zeit keine Veränderungen.

### Beispiel 2 (Vergleich)

Die Hydrierung des ε-Caprolactams erfolgt in der Apparatur und an dem Kupferkatalysator des Beispiels 1 bei 250°C, 3 bar H$_2$-Druck unter Einsatz von 30 g/h ε-Caprolactam und 150 l/h H$_2$, jedoch ohne Zusatz von NH$_3$.

Nach Koindensation wird ein Reaktionsprodukt folgender Zusammensetzung erhalten:

57 Gew.-% Hexamethylenimin
27 Gew.-% Nebenprodukte
1 Gew.-% ε-Caprolactam
15 Gew.-% Wasser

Innerhalb von 28 Betriebstagen stellt man einen gleichmäßigen Rückgang des ε-Caprolactam-Umsatzes von etwa 99 % auf etwa 50 % fest.

### Beispiel 3

Ein weiterer Versuch zur Hydrierung von ε-Caprolactam wird in der Apparatur des Beispiels 1 und mit dem gleichen Katalysator unter Aufgabe von 35 g/h ε-Caprolactam, 150 l/h H$_2$ und 5 l/h NH$_3$ bei 250°C durchgeführt. Die Hydrierung erfolgt bei einem Reaktionsdruck von 4 bar. Das kondensierte Reaktinsprodukt hat folgende Zusammensetzung:

67 Gew.-% Hexamethylenimin
3 Gew.-% Nebenprodukte
15 Gew.-% ε-Caprolactam
15 Gew.-% Wasser

### Beispiel 4

Ein weiterer Versuch zur Hydrierung von ε-Caprolactam wird in der gleichen Aparatur und mit dem gleichen Katalysator wie in Beispiel 1 unter geändertem Reaktionsdruck von 6 bar durchgeführt. Bei Einsatz von 30 g/h ε-Caprolactam, 150 l/h H$_2$ und 3 l/h NH$_3$ wird bei einer Reaktionstemperatur von 250°C ein Reaktionsprodukt folgender Zusammensetzung erhalten:
69 Gew.-% Hexamethylenimin
4 Gew.-% Nebenprodukte
14 Gew.-% ε-Caprolactam
13 Gew.-% Wasser

### Patentansprüche

1. Verfahren zur Herstellung von Hexamethylenimin durch Umsetzung von ε-Caprolactam mit Wasserstoff in der Gasphase an einem als Festbett oder als Wirbelschicht angeordneten Katalysator, dadurch gekennzeichnet, daß die Hydrie-

rung des ε-Caprolactams bei Temperaturen zwischen 200 und 350°C, vorzugsweise zwischen 220 bis 300°C und bei Atmosphärendruck oder Überdruck bis 10 bar, vorzugsweisie bis 5 bar, durchgeführt wird und in Gegenwart von Kupfer-Trägerkatalysatoren mit einem Wasserstoff-Ammoniak-Gemisch, das 0,5 bis 20 Vol.-% Ammoniak, bezogen auf das Wasserstoff-Ammoniak-Gemisch enthält, erfolgt.

2. Verfahren nach Anspruch 1, dadurch gekennzeichnet, daß die Konzentration des Ammoniaks im Wasserstoff-Ammoniak-Gemisch 1 bis 5 Vol.-% beträgt.

3. Verfahren nach Anspruch 1 und 2, dadurch gekennzeichnet, daß die Verweilzeit der gasfömigen Reaktanten unter den Reaktionsbedingungen in dem vom Katalysator eingenommenen Reaktorvolumen 2 bis 20 Sekunden beträgt.

## Claims

A process for the preparation of hexamethyleneimine by the reaction of ε-caprolactam with hydrogen in the gaseous phase on a fixed-bed or fluidised bed catalyst, characterised in that the ε-caprolactam is hydrogenated at temperatures between 200 and 350°C, preferably between 220 and 30°C and at atmospheric pressure or at an excess pressure of up to 10 bar, preferably up to 5 bar, and takes place in the presence of copper carrier catalysts with a hydrogen-ammonia mixture containing 0.5 to 20 mol-% ammonia, related to the hydrogen-ammonia mixture.

2. A process according to claim 1, characterised in that the ammonia concentration in the hydrogen-ammonia mixture amounts to 1 to 5 vol-%.

3. A process according to claims 1 and 2, characterised in that the residence period of the gaseous reactants under the reaction conditions in that part of the reactor volume occupied by the catalyst amounts to 2 to 20 seconds.

## Revendications

1. Procédé pour préparer l'hexaméthylène-imine par réaction de l'ε-caprolactame avec l'hydrogène en phase gazeuse sur un catalyseur disposé sous forme de lit solide ou de couche tourbillonnante, caractérisé en ce qu'on conduit l'hydrogénation de l'ε-caprolactame à des températures comprises entre 200 et 350°C, avantageusement entre 220 et 300°C et à la pression atmosphérique ou sous une surpression allant jusqu'à 10 bars, avantageusement jusqu'à 5 bars, et en ce qu'on opère en présence de catalyseurs au cuivre sur support avec un mélange hydrogène/ammoniac, qui contient 0,5 à 20% en volume d'ammoniac, par rapport au mélange hydrogène/ammoniac.

2. Procédé selon la revendication 1, caractérisé en ce que la concentration de l'ammoniac est de 1 à 5% en volume dans le mélange hydrogène/ammoniac.

3. Procédé selon la revendication 1 et la revendication 2, caractérisé en ce que le temps de séjour des corps gazeux desinés à réagir dans les conditions de réaction est de 2 à 20 s dans le volume de réacteur occupé par le catalyseur.